# EUROPÄISCHE PATENTSCHRIFT

(11) **EP 3 230 253 B1**
(45) Veröffentlichungstag und Bekanntmachung des Hinweises auf die Patenterteilung: **25.12.2019**
(21) Anmeldenummer: 15802089.1
(22) Anmeldetag: 30.11.2015
(51) Int. Cl.: C07C 67/54, C07C 69/24, C07C 69/52

(54) **VERFAHREN ZUR REINIGUNG VON FETTSÄUREALKYLESTERN**
METHOD FOR PURIFYING FATTY ACID ALKYL ESTERS
PROCÉDÉ DE NETTOYAGE D'ESTERS ALKYLES D'ACIDES GRAS

(30) Priorität: 11.12.2014 EP 14197324
(43) Veröffentlichungstag der Anmeldung: 18.10.2017
(73) Patentinhaber: BDI Holding GmbH, 8074 Grambach (AT)
(72) Erfinder: REICHEL, Martin, 8010 Graz (AT); RAUDNER, Robert, 8580 Köflach (AT)
(74) Vertreter: Bachinger-Fuchs, Eva-Maria
(86) Internationale Anmeldenummer: PCT/EP2015/078085
(87) Internationale Veröffentlichungsnummer: WO 2016/091640

(56) Entgegenhaltungen:
- WO-A1-99/24387
- WO-A1-2004/083350
- Patricia Bogalhos Lucente Fregolente ET AL: "Water Content in Biodiesel, Diesel, and Biodiesel-Diesel Blends", JOURNAL OF CHEMICAL AND ENGINEERING DATA., vol. 57, no. 6, 17 May 2012 (2012-05-17), pages 1817-1821, XP055494397, US ISSN: 0021-9568, DOI: 10.1021/je300279c

## Beschreibung

Die Erfindung betrifft ein Verfahren zur Reinigung von Fettsäurealkylestern, inbesondere Methyl- und Ethylestern, mittels Vakuumdestillation in einer Destillationskolonne.

Für die Produktion von Biodiesel (Fettsäuremethylester = FAME) nach der europäischen Norm EN 14214 werden im industriellen Maßstab Pflanzenöle oder Abfallprodukte, wie Altspeiseöle, Spaltfettsäuren oder tierische Fette, verwendet. Insbesondere Tierfett der Kategorie 1, ein Hochrisikomaterial bezüglich der Kontamination mit TSE (Transmissible Spongiforme Enzephalopathie, z.B. BSE), kann mittels der chemischen Umwandlung in einen Fettsäuremethylester mit anschließender Destillation in einen wertvollen Treibstoff umgewandelt werden.

Ein wesentlicher Nachteil dieser Ausgangsstoffe, insbesondere der Abfallstoffe Spaltfettsäuren und Tierfett, besteht allerdings in deren hohen Anteil an Schwefel, der bei Spaltfettsäuren aus dem Aufarbeitungsprozess und bei Tierfetten aus dem tierischen Ausgangsmaterial stammt. Im fertigen Produkt, also Biodiesel, ist der Schwefelgehalt gemäß EN 14214 mit maximal 10 ppm reglementiert. Es hat sich jedoch gezeigt, dass selbst nach der Umesterung noch Schwefelverbindungen im Ester nachweisbar sind, so dass der Grenzwert von 10 ppm fallweise bei weitem überschritten wird. Das üblicherweise vorgenommene Waschen des Rohesters und eine daran anschließende Destillation führen auch nur zu einer mäßigen Senkung des Schwefelgehalts, so dass dem Ausgangsmaterial in Bezug auf die Schwefelkonzentration Grenzen gesetzt sind.

Um Rohstoffe mit höheren Schwefelgehalten verarbeiten zu können, muss die Destillation überdimensioniert und/oder mit weniger Last bzw. größerer Rückstandsrate gefahren werden, was jedoch zu höheren Betriebskosten, schlechterer Ausbeute und geringem Durchsatz führt sowie höhere Investitionen bedingt.

Um den Anteil an Schwefel im Biodiesel zu reduzieren, sind daher verschiedene Verfahren vorgeschlagen worden:
Gemäß der US 8,585,901 werden die Rohstoffe für die Biodiesel-Produktion mit schwefeloxidierenden Mikroben, z.B. *Thiobacillus* sp. und *Alcaligenes* sp., behandelt, um den Schwefelgehalt vor der Veresterung zu reduzieren.

Die WO 2004/083350 lehrt ein Verfahren, bei dem der gewonnene Fettsäurealkylester mit einem basischen Medium, z.B. KOH oder CaOH, behandelt wird.

Gemäß der US 6,242,620 werden die Ester mittels wässriger Lauge und Silicagel gereinigt.

In der US 2013/0212933 wird ein Verfahren zur Herstellung von schwefelarmem Biodiesel aus tierischen und pflanzlichen Ölen beschrieben, bei dem die Rohstoffe einer Veresterung mittels reaktiver Destillation unterzogen werden, wobei bei der reaktiven Destillation ein sulfonsäurehältiger Ionenaustauscher als Katalysator eingesetzt wird.

Ein ähnliches Verfahren wird auch in der WO 2009/018390 beschrieben, wobei im Unterschied zur US 2013/0212933 vor der Veresterung eine Vakuumdestillation zur Vorreinigung der freien Fettsäuren durchgeführt wird. Nach der Veresterung kann das flüssige Rohprodukt noch einem zusätzlichen Reinigungsschritt, zB. einer Destillation, einer Adsorption oder Reboiled Stripping, unterzogen werden.

Bei den bekannten Verfahren wird zwar eine Reduktion des Schwefelgehalts erzielt, doch ist diese meist nicht ausreichend oder erfordert zusätzlichen Aufwand, um auch stark schwefelhältige und damit kostengünstige Ausgangsstoffe einsetzen zu können.

Die Erfindung stellt sich daher die Aufgabe, ein Verfahren zur Reinigung von Fettsäurealkylestern bereitzustellen, das eine Verwendung von stark schwefelhältigen Rohstoffen unter Einhaltung des Schwefelgrenzwerts von 10 ppm gemäß EN 14214 ermöglicht, ohne dass eine unvorteilhafte Prozessführung oder zusätzliche Reinigungsschritte erforderlich sind.

Diese Aufgabe wird bei einem Verfahren der eingangs genannten Art erfindungsgemäß dadurch gelöst, dass in die Destillationskolonne Wasser oder Wasserdampf eingebracht und bei der Destillation mit den Fettsäurealkylestern in der Gasphase kontaktiert wird, wobei die leichtflüchtigen Komponenten einschließlich Wasser vor der Destillation aus den Fettsäurealkylestern entfernt werden und das Wasser oder der Wasserdampf in einer Menge von 0,5-10,0 kg/t Fettsäurealkylester, vorzugsweise 2,0-5,0 kg/t Fettsäurealkylester, in die Destillationskolonne eingebracht wird.

Da sich Restfeuchtigkeit im Destillationsgut im Allgemeinen nachteilig auf die Destillation, insbesondere deren Vakuumsystem, auswirkt, wird das Wasser üblicherweise vor der Destillation durch geeignete Maßnahmen, z.B. eine Trocknungkolonne, aus den Fettsäurealkylestern entfernt. Überraschenderweise konnte jedoch festgestellt werden, dass es zu einer signifikanten Reduktion des Schwefelgehalts kommt, wenn der Fettsäurealkylester, also z.B. der Rohbiodiesel, während der Destillation, d.h. während er sich in der Gasphase befindet, mit Wasserdampf in Kontakt gebracht wird. Es hat sich gezeigt, dass besonders jene Schwefelverbindungen entfernt werden können, die sich mittels einer herkömmlichen Destillation nicht weiter abtrennen lassen.

Ein weiterer überraschender Vorteil des erfindungsgemäßen Verfahrens besteht darin, dass neben der Reduktion des Schwefelgehalts auch eine Reduktion der Neutralisationszahl (= mg Kaliumhydroxid, die notwendig sind, um die in 1 g Probe enthaltenen Säuren zu neutralisieren) erzielt wird. Insbesondere für Biodiesel ist eine niedrige Neutralisationszahl bzw. Säurezahl (Grenzwert gemäß EN 14214: 0,5 mg KOH/g), d.h. ein niedriger Gehalt an freien Fettsäuren, wünschenswert, weil saure Verbindungen im Kraftstoff zu Korrosion, Verschleiß und Rückstandsbildung im Motor führen.

Zur Erzielung der erforderlichen Schwefelreduktion wird in die Destillationskolonne Wasser oder Wasserdampf in einer Menge von 0,5-10,0 kg/t Fettsäurealkylester, bevorzugt 2,0-5,0 kg/t Fettsäurealkylester, eingebracht. Unterhalb von 0,5 kg/t findet keine wirtschaftlich sinnvolle reinigende Wirkung mehr statt, wohingegen es oberhalb von 10,0 kg/t zu keiner nennenswerten Steigerung des Reinigungseffekts sondern im Gegenteil durch den Wassereintrag eher zu einer operativen Beeinträchtigung der Vakuumdestillation kommt.

Gemäß einer bevorzugten Ausführungsform wird das Wasser oder der Wasserdampf einem Teil des Destillationsrückstands zugeführt, welcher in die Destillationskolonne rückgeführt wird, so dass das Wasser gemeinsam mit diesem Destillationsrückstand in die Destillationskolonne eingebracht wird.

Gemäß einer weiteren bevorzugten Ausführungsform wird das Wasser oder der Wasserdampf den zu reinigenden Fettsäurealkylestern zugeführt und gemeinsam mit diesen in die Destillationskolonne eingebracht.

Eine weitere bevorzugte Ausführungsform ist dadurch gekennzeichnet, dass das Wasser oder der Wasserdampf direkt in die Destillationskolonne eingespeist wird.

Zwar kann die Zudosierung von Wasser oder Wasserdampf hierbei an jedem Punkt vor der eigentlichen Destillation, also dem Abtrennen der leichterflüchtigen Komponenten, erfolgen, doch ist die Zufuhr im unteren Teil der Destillationskolonne, z.B. in den Fallfilmverdampfer, von besonderem Vorteil, da es hierdurch zu einem besonders langen Kontakt zwischen Wasser/Wasserdampf und gasförmiger Esterphase kommt.

Die leichtflüchtigen Komponenten einschließlich Wasser werden vor der Destillation aus den Fettsäurealkylestern entfernt. Hierzu wird vorzugsweise eine Flashverdampfung durchgeführt.

Durch diese Maßnahme gelingt es, konstante Bedingungen in der nachfolgenden Vakuumdestillation herbeizuführen, da durch eine vorherige Abtrennung von Restfeuchtigkeit aus dem Rohester eine mengenmäßig kontrollierte Zugabe von Wasser oder Wasserdampf in einfacher Weise ermöglicht wird.

Außerdem wirkt sich diese Maßnahme positiv auf die nachfolgende Vakuumdestillation aus, da durch die Entfernung der im Esterrohprodukt noch vorhandenen leichtflüchtigen Komponenten, wie z.B. Methanol, die Dimensionierung des Vakuumsystems geringer ausfallen kann.

Bevorzugt wird die Vakuumdestillation bei einem Druck < 10 mbar, vorzugsweise < 3mbar, durchgeführt. Die bevorzugte Temperatur für die Vakuumdistillation beträgt 100-260°C, vorzugsweise 140-200°C.

Die Vakuumdestillation kann einstufig, z.B. mittels Kurzwegdestillation, oder mehrstufig über eine Rektifikation erfolgen. In jedem Fall ist es jedoch vorteilhaft, bei der Zufuhr von Wasser oder Wasserdampf darauf zu achten, dass die Kontaktzeiten zwischen Wasser und Esterprodukt in der Gasphase maximiert werden.

Die Erfindung wird nachfolgend anhand eines Beispiels sowie der Zeichnung näher erläutert.

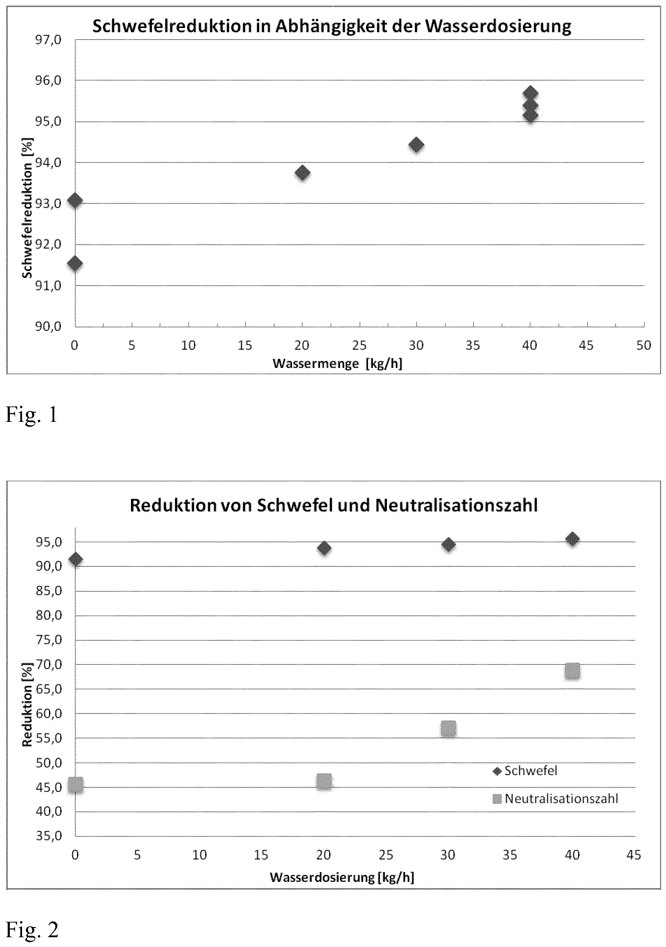

**Fig. 1** zeigt eine graphische Darstellung der Schwefelreduktion in Abhängigkeit der Menge an zudosiertem Wasser bei einer Vakuumdestillation gemäß einer Ausführungsform der Erfindung.
**Fig. 2** zeigt eine graphische Darstellung der Schwefel- sowie der Säurezahlreduktion in Abhängigkeit der Menge an zudosiertem Wasser bei einer Vakuumdestillation gemäß einer Ausführungsform der Erfindung.

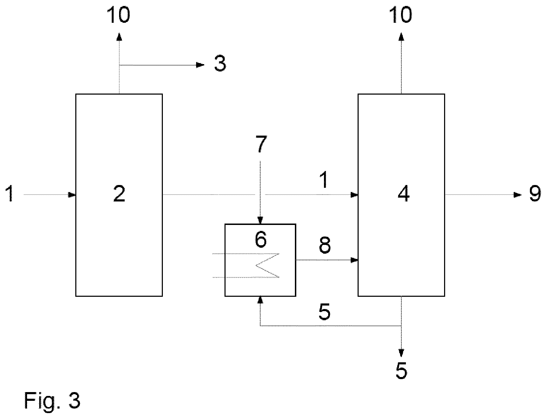

**Fig. 3** zeigt ein Blockdiagramm einer Ausführungsform des erfindungsgemäßen Verfahrens.

### Beispiele

Die Auswirkungen des Einbringens von Wasser oder Wasserdampf in die Destillationskolonne, um den Rohbiodiesel in der Gasphase mit Wasser bzw. Wasserdampf während der Vakuumdestillation zu kontaktieren, wurden untersucht.

Vor dem Beginn der Untersuchung wurden die Betriebskenngrößen, wie Massenbilanz, Rückflussgeschwindigkeiten, Zufuhrströme, etc., der Destillationskolonne (Rektifikationssäule) ermittelt. Nach dem Einstellen konstanter Bedingungen wurden Proben des Ausgangsprodukts bzw. Zufuhrstroms und des Destillats genommen und bezüglich ihres Schwefelgehalts sowie der Neutralisationszahl analysiert.

Im ersten Schritt wurde der Rohbiodiesel 1 in einer Flashkolonne 2 von leichtflüchtigen Substanzen 3 einschließlich Wasser befreit und anschließend in die Destillationskolonne 4 eingespeist. Die mittlere Zustromrate von der Flashkolonne 2 zur Destillationskolonne 4 betrug während der Versuche 19,5 t/h. Der Druck bzw. das Vakuum an der Spitze der Säule betrug zwischen 1,0 und 3,1 mbar. Die mittlere Destillationsrückstandsrate des Destillationssystems wurde bei 5,4 % gehalten. Aufgrund der erwarteten Zunahme des Dampfdrucks in der Destillationssäule wurde eine höhere Sumpftemperatur (184°C-189°C) eingestellt, um eine Destillationsrückstandsrate wie im Normalbetrieb (d.h. ohne Wasser/Dampfzufuhr) zu erhalten.

Die zur Destillation notwendige Energie wurde über einen Sumpfkreislaufstrom zugeführt. Hierbei wurde ein Teil des aus dem Kolonnensumpf abgezogenen Destillationsrückstands 5 in einem Wärmetauscher 6 erhitzt. Im Wärmetauscher 6 wurde auch Wasser 7 zudosiert und so dem in die Kolonne rückzuführenden Teil des Destillationsrückstands 5 zugeführt. Das erhitzte Gemisch 8 aus Destillationsrückstand 5 und Wasser 7 wurde dann in die Destillationskolonne 4 eingebracht, in der es hierauf bei der Destillation zu einem innigen Kontakt zwischen dem Wasser bzw. Wasserdampf und dem gasförmigen Rohbiodiesel 1 kam.

Der gereinigte Biodiesel 9 wurde aus der Destillationskolonne 4 abgezogen und analysiert. Die leichtflüchtigen Schwefelverbindungen sowie das in die Kolonne eingebrachte Wasser wurden durch das Vakuumsystem 10 entfernt. Der wesentliche Anteil des "ausgewaschenen" Schwefels wurde aus dem Kolonnensumpf mit dem Destillationsrückstand 5 abgezogen und aus dem Sumpfkreislaufstrom ausgeschleust.

Tabelle 1 zeigt die Ergebnisse für den Normalbetrieb (Versuch Nr. 9A) sowie für die Einbringung unterschiedlicher Mengen von Wasser in die Destillationskolonne.

**Tabelle 1:**

| **Versuch Nr.** | **Wasserzudosierung** | | **S-Gehalt (Ausgangsprodukt)** | **S-Gehalt (nach Destillation)** | **S-Reduktion** |
|---|---|---|---|---|---|
| | **[kg/h]** | **[kg H₂O/t Ester]** | **[ppm]** | | **[%]** |
| 9A | 0 | 0 | 130 | 11 | 91,5 |
| 14 | 20 | 1,08 | 128 | 8 | 93,8 |
| 11 | 30 | 1,54 | 126 | 7 | 94,4 |
| 8 | 40 | 2,22 | 152 | 7 | 95,4 |
| 9 | 40 | 2,34 | 124 | 6 | 95,2 |
| 10 | 40 | 2,05 | 139 | 6 | 95,7 |

Tabelle 2 führt neben den Ergebnissen für den Schwefelgehalt auch die Ergebnisse der Säurezahlanalyse an.

**Tabelle 2:**

| **Versuch Nr.** | **Wasserzudosierung** | **Säurezahl (Ausgangsprodukt)** | **Säurezahl (nach Destillation)** | **Säurezahl-Reduktion** |
|---|---|---|---|---|
| | **[kg/h]** | **[mg KOH/g Ester]** | | **[%]** |
| 9A | 0 | 1,03 | 0,56 | 45,6 |
| 14 | 20 | 0,97 | 0,52 | 46,4 |
| 11 | 30 | 0,91 | 0,39 | 57,1 |
| 10 | 40 | 1,22 | 0,38 | 68,9 |

Die Ergebnisse zeigen, dass das Einbringen von Wasser/Dampf in die Destillationskolonne zu einer signifikanten Verbesserung führt. Trotz der erforderlichen höheren Verdampfungstemperatur im Säulensumpf aufgrund des höheren Systemdrucks konnten der Schwefelgehalt und überraschenderweise auch die Neutralisationszahl des Biodiesels deutlich reduziert werden.

## Patentansprüche

1. Verfahren zur Reinigung von Fettsäurealkylestern, insbesondere Methyl- und Ethylestern, mittels Vakuumdestillation in einer Destillationskolonne, **dadurch gekennzeichnet, dass** in die Destillationskolonne Wasser oder Wasserdampf eingebracht und bei der Destillation mit den Fettsäurealkylestern in der Gasphase kontaktiert wird, wobei die leichtflüchtigen Komponenten einschließlich Wasser vor der Destillation aus den Fettsäurealkylestern entfernt werden und das Wasser oder der Wasserdampf in einer Menge von 0,5-10,0 kg/t Fettsäurealkylester, vorzugsweise 2,0-5,0 kg/t Fettsäurealkylester, in die Destillationskolonne eingebracht wird.

2. Verfahren nach Anspruch 1, **dadurch gekennzeichnet, dass** der Destillationsrückstand teilweise in die Destillationskolonne rückgeführt wird und das Wasser oder der Wasserdampf dem rückzuführenden Teil des Destillationsrückstands zugeführt und gemeinsam mit diesem in die Destillationskolonne eingebracht wird.

3. Verfahren nach Anspruch 1, **dadurch gekennzeichnet, dass** das Wasser oder der Wasserdampf den zu reinigenden Fettsäurealkylestern zugeführt und gemeinsam mit diesen in die Destillationskolonne eingebracht wird.

4. Verfahren nach Anspruch 1, **dadurch gekennzeichnet, dass** das Wasser oder der Wasserdampf direkt in die Destillationskolonne eingespeist wird.

5. Verfahren nach einem der Ansprüche 1-4, **dadurch gekennzeichnet, dass** die leichtflüchtigen Komponenten einschließlich Wasser mittels Flashverdampfung entfernt werden.

6. Verfahren nach einem der Ansprüche 1 bis 5, **dadurch gekennzeichnet, dass** die Vakuumdestillation bei einem Druck < 10 mbar, vorzugsweise < 3mbar, durchgeführt wird.

7. Verfahren nach einem der Ansprüche 1 bis 6, **dadurch gekennzeichnet, dass** die Vakuumdestillation bei einer Temperatur von 100-260°C, vorzugsweise 140-200°C, durchgeführt wird.

## Claims

1. A process for purifying fatty acid alkyl esters, particularly methyl and ethyl esters, by means of vacuum distillation in a distillation column, **characterized in that** water or steam is introduced into the distillation column and, during distillation, is brought into contact with the fatty acid alkyl esters in the gas phase, wherein the highly volatile components including water are removed from the fatty acid alkyl esters prior to distillation and the water or steam is introduced in an amount of 0.5-10.0 kg/t of fatty acid alkyl ester, preferably 2.0-5.0 kg/t of fatty acid alkyl ester, into the distillation column.

2. A process according to claim 1, **characterized in that** the distillation residue is partially returned into the distillation column and the water or steam is supplied to the part of the distillation residue to be returned and introduced together with said part into the distillation column.

3. A process according to claim 1, **characterized in that** the water or steam is supplied to the fatty acid alkyl esters to be purified and introduced together with said esters into the distillation column.

4. A process according to claim 1, **characterized in that** the water or steam is fed directly into the distillation column.

5. A process according to any of claims 1 to 4, **characterized in that** the highly volatile components including water are removed through flash evaporation.

6. A process according to any of claims 1 to 5, **characterized in that** the vacuum distillation is carried out at a pressure of < 10 mbar, preferably < 3 mbar.

7. A process according to any of claims 1 to 6, **characterized in that** the vacuum distillation is carried out at a temperature of 100-260°C, preferably 140-200°C.

## Revendications

1. Procédé de nettoyage d'esters alkyles d'acides gras, en particulier d'esters méthyliques et d'esters éthyliques, par distillation sous vide dans une colonne de distillation, **caractérisé en ce que** l'on introduit de l'eau ou de la vapeur d'eau dans la colonne de distillation et on la met en contact lors de la distillation avec les esters alkyles d'acides gras en phase gazeuse, dans lequel on élimine les composants aisément volatils y compris l'eau avant la distillation hors des esters alkyles d'acides gras et on introduit l'eau ou la vapeur d'eau en une quantité de 0,5 - 10,0 kg/t d'ester alkyle d'acides gras, de préférence de 2,0 - 5,0 kg/t d'ester alkyle d'acides gras, dans la colonne de distillation.

2. Procédé selon la revendication 1, **caractérisé en ce que** l'on renvoie le résidu de distillation en partie dans la colonne de distillation et on ajoute l'eau ou la vapeur d'eau à la partie du résidu de distillation à renvoyer et on l'introduit ensemble avec celui-ci dans la colonne de distillation.

3. Procédé selon la revendication 1, **caractérisé en ce que** l'on ajoute l'eau ou la vapeur d'eau aux esters alkyles d'acides gras à nettoyer et on l'introduit ensemble avec ceux-ci dans la colonne de distillation.

4. Procédé selon la revendication 1, **caractérisé en ce que** l'on charge l'eau ou la vapeur d'eau directement dans la colonne de distillation.

5. Procédé selon l'une quelconque des revendications 1 à 4, **caractérisé en ce que** l'on élimine les composants aisément volatils y compris l'eau au moyen d'une évaporation flash.

6. Procédé selon l'une quelconque des revendications 1 à 5, **caractérisé en ce que** l'on effectue la distillation sous vide sous une pression < 10 mbar, de préférence < 3 mbar.

7. Procédé selon l'une quelconque des revendications 1 à 6, **caractérisé en ce que** l'on effectue la distillation sous vide à une température de 100 - 260°C, de préférence de 140 - 200°C.
